# EUROPEAN PATENT APPLICATION

(11) **EP 4 691 412 A1**
(43) Date of publication of application: **11.02.2026**
(21) Application number: 24825048.2
(22) Date of filing: 14.05.2024
(51) Int. Cl.: A61B 90/30

(54) **ILLUMINATION APPARATUS FOR MEDICAL INSTRUMENT AND MEDICAL INSTRUMENT KIT**

(30) Priority: 21.06.2023 CN 202310749306
(71) Applicant: SMTP Medical Co., Ltd., Beijing 102629 (CN)
(72) Inventor: CAO, Qun, Beijing 102629 (CN); ZHAN, Songtao, Beijing 102629 (CN); SHI, Junqiang, Beijing 102629 (CN); DAI, Zhiling, Beijing 102629 (CN)
(74) Representative: Reddie & Grose LLP
(86) International application number: PCT/CN2024/093056
(87) International publication number: WO 2024/260151

(57) **Abstract**

The present disclosure provides an illumination apparatus for a medical instrument, and a medical instrument kit. The illumination apparatus for a medical instrument includes: a housing component provided with an accommodating space that runs along a longitudinal axis of the housing component; and an illumination assembly disposed within the accommodating space and provided with a channel that runs along the longitudinal axis. The illumination apparatus is capable of providing illumination while using the medical instrument, enabling a person operating the medical instrument to have a better vision.

## Description

### CROSS REFERENCE

The present disclosure refers to Chinese Patent Application No. 202310749306.6, filed on June 21, 2023 and entitled "ILLUMINATION APPARATUS FOR MEDICAL INSTRUMENT AND MEDICAL INSTRUMENT KIT", which is incorporated herein by reference in its entirety.

### TECHNICAL FIELD

The present disclosure relates to the technical field of medical instruments, and in particular to an illumination apparatus for a medical instrument, and a medical instrument kit.

### BACKGROUND ART

Currently, illumination in surgical equipment mainly relies on shadowless lamps, which are generally effective in most application scenarios. However, in some procedures, it is difficult to achieve illumination, as light cannot reach a deep location of a wound, which is particularly pronounced in scenarios involving deep, narrow wounds with substantial internal cavities, making it inconvenient to use.

### SUMMARY OF THE INVENTION

The present disclosure is intended to solve at least one of the technical problems existing in the background art.

According to an aspect of the present disclosure, some embodiments of the present disclosure provide an illumination apparatus for a medical instrument, the illumination apparatus including: a housing component provided with an accommodating space that runs along a longitudinal axis of the housing component; and an illumination assembly disposed within the accommodating space and provided with a channel that runs along the longitudinal axis.

By applying the illumination apparatus according to some embodiments of the present disclosure to a medical instrument, such as by securing the illumination apparatus to the medical instrument, direct illumination can be provided to an operating site during operation of the medical instrument, thereby facilitating a clear view of the operating site by an operator of the medical instrument.

It should be understood that "medical instrument" in the present disclosure may include any type of medical instruments, such as a scalpel, a surgical clamp, surgical forceps, an irrigation or suction tubing, etc. The scalpel may include an ultrasonic scalpel and an electrosurgical scalpel.

In some embodiments according to the present disclosure, the illumination assembly may include: a light-emitting element; and a light-guiding element disposed adjacent to the light-emitting element, the light-guiding element being configured to guide light emitted from the light-emitting element to the outside of the illumination apparatus; where the light-emitting element and the light-guiding element together define a channel.

In the above embodiments of the present disclosure, the light-emitting element may be disposed abutting against the light-guiding element or may be spaced apart from the light-guiding element by a distance so long as the light-guiding element is capable of guiding the light emitted from the light-emitting element to the outside of the illumination apparatus.

In some embodiments according to the present disclosure, the light-emitting element may be annular, and the light-guiding element may also be cylindrical, thus the light-emitting element and the light-guiding element can together define a channel having a circular cross-section.

In some embodiments according to the present disclosure, the light-emitting element includes any one or a combination of two or more of a halogen lamp, an LED emitter, or an optical fiber.

In some embodiments according to the present disclosure, the light-guiding element is transparent or translucent.

In some embodiments according to the present disclosure, the light-guiding element is made of any one or more materials selected from polycarbonate, polyethylene terephthalate, polypropylene, and polymethyl methacrylate.

In some embodiments according to the present disclosure, the light-guiding element further includes a light diffusion agent.

The light diffusion agent may include an inorganic light diffusion agent (e.g., nano barium sulfate, calcium carbonate, silicon dioxide, etc.) and an organic light diffusion agent (e.g., acrylic-based, styrene-based, acrylic resin-based, etc.). By adding the light diffusion agent to the light-guiding element, an illumination range and/or uniformity of illumination of the illumination apparatus can be expanded.

In some embodiments according to the present disclosure, the light-guiding element extends by a predetermined distance from the accommodating space.

Since an outer dimension of the light-guiding element is smaller than an outer dimension of the housing component, the light-guiding element may be brought closer to the operating site of the medical instrument by arranging the light-guiding element to extend by a predetermined distance from within the accommodating space, and it also avoids obstructing the view of the operator of the medical instrument as the housing component having a larger outer dimension is close to the operating site of the medical instrument.

In some embodiments according to the present disclosure, the illumination apparatus further includes a power supply electrically connected to the illumination assembly.

It will be appreciated that the "power supply" herein may be a power supply fixedly connected to the illumination assembly, or may be a power supply detachably connected to the illumination assembly (e.g., the power supply is an external power supply that is electrically connected to the illumination assembly by a wire).

In some embodiments according to the present disclosure, the housing component is provided with a power supply compartment for accommodating the power supply and a cover for enclosing the power supply compartment.

In some embodiments according to the present disclosure, the cover may hermetically enclose the power supply in the power supply compartment.

Since the medical instrument may be in direct contact with a liquid such as a body fluid (such as blood) or an irrigation fluid during use, the illumination apparatus may also be in direct contact with the body fluid (such as blood) or the irrigation fluid. By hermetically accommodating the power supply in the power supply compartment, it is possible to avoid short-circuiting of the interior of the illumination apparatus by these liquids.

In some embodiments according to the present disclosure, the illumination apparatus further includes a switch disposed in the housing component, the switch being configured to selectively turn on and off an electrical connection between the power supply and the illumination assembly.

By setting the above switch, it is possible to selectively turn on and off the electrical connection between the power supply and the illumination assembly during use of the medical instrument.

In some embodiments according to the present disclosure, the housing component has a first end and a second end opposite each other, the light-guiding element is disposed adjacent to the first end, and the switch is disposed adjacent to the second end.

In order to better illuminate the operating site of the medical instrument, during use, the end of the light-guiding element for illumination should be as close as possible to the operating site of the medical instrument, and for a procedure with a deeper wound, the end of the light-guiding element for illumination may enter the wound. By placing the switch as far away from the light-guiding element as possible, the switch can be easily operated.

In some embodiments according to the present disclosure, the switch is a disposable switch.

In order to avoid possible infections caused by reuse, illustratively, the illumination apparatus in the embodiments of the present disclosure may be a disposable illumination apparatus. In order to prevent the illumination apparatus of the present disclosure from being undesirably reused, the switch may be set as a disposable switch, i.e., the switch cannot be turned off again once turned on, or the switch cannot be turned on again once it is turned on and off once.

In some embodiments according to the present disclosure, the illumination apparatus further includes a disposable insulation member that is configured to disconnect an electrical connection between the power supply and the illumination assembly.

In the above embodiments of the present disclosure, the disposable insulation member described above can function as a switch, i.e., by removing the insulation member, the electrical connection between the power supply and the illumination assembly can be established, and the illumination apparatus is thus used for illumination. Since the insulation member is a disposable insulation member, that is, once the insulation member is removed from the illumination apparatus, the insulation member cannot be used again to disconnect the electrical connection between the power supply and the illumination assembly, the insulation member also prevents the illumination apparatus from being reused.

In the case where the illumination apparatus of the present disclosure is itself provided with a switch that selectively turns on and off the electrical connection between the power supply and the illumination assembly, the insulation member may be configured to prevent the illumination apparatus from inadvertently touching the switch during transport or use to establish an electrical connection between the power supply and the illumination assembly. That is, before the disposable insulation member is removed, even if the switch is on, the electrical connection between the power supply and the illumination assembly is also disconnected by the insulation member.

In some embodiments according to the present disclosure, the housing component includes a connection for detachably securing the illumination apparatus to the medical instrument.

The above embodiments of the present disclosure are particularly applicable to reusable medical instruments. That is, after the medical instrument is used, the illumination apparatus can be removed from the medical instrument to facilitate operations such as disinfection of the medical instrument.

According to another aspect of the present disclosure, some embodiments of the present disclosure provide a medical instrument kit, including: a medical instrument; and an illumination apparatus according to any of the above embodiments.

It should be understood that according to the medical instrument kit of the above embodiments of the present disclosure, the illumination apparatus may be fixedly disposed on the medical instrument, the illumination apparatus and the medical instrument may be disposed separate from each other, and the illumination apparatus is fixed to the medical instrument only when the medical instrument is in use.

In some embodiments according to the present disclosure, a portion of the medical instrument is accommodated within a channel of an illumination assembly.

The illumination apparatus is configured to illuminate an operating site of the medical instrument so that an operator of the medical instrument can clearly see the operating site. Thus, illustratively, the illumination apparatus is disposed on the medical instrument in proximity to the operating site of the medical instrument to better illuminate the operating site of the medical instrument. As the portion of the medical instrument is accommodated within the channel of the illumination assembly, certain components of the medical instrument can be prevented from blocking light emitted by the illumination apparatus, while 360-degree omnidirectional illumination around the medical instrument is also provided.

In some embodiments according to the present disclosure, the illumination apparatus is detachably fixed to the medical instrument.

As mentioned above, the above embodiments of the present disclosure are particularly applicable to reusable medical instruments. That is, after the medical instrument is used, the illumination apparatus can be removed from the medical instrument to facilitate operations such as disinfection of the medical instrument.

In some embodiments according to the present disclosure, the medical instrument includes a handle, and the illumination apparatus is detachably connected to the handle.

The above embodiments of the present disclosure are particularly applicable to ultrasonic medical instruments, such as various types of ultrasonic scalpels. The ultrasonic medical instruments include vibration sources (such as ultrasound transducers, which can be used as handles for the ultrasonic medical instruments) and actuation components (configured to amplify vibrations generated by the vibration sources for surgical operations). By securing the illumination apparatus to the handle, it is possible to prevent the illumination apparatus from affecting the operation of the actuation component of the ultrasonic medical instrument, and it is also possible to prevent vibration of the actuation component from affecting an illumination effect of the illumination apparatus.

In some embodiments according to the present disclosure, the illumination apparatus is detachably connected to the handle by a threaded connection or a snap connection or an interference fit.

In some embodiments according to the present disclosure, the illumination apparatus is detachably fixed to the medical instrument by an interference fit of the portion of the medical instrument with the channel of the illumination assembly.

In some embodiments according to the present disclosure, a gap is provided between the portion of the medical instrument and the channel.

The above embodiments of the present disclosure are also particularly applicable to ultrasonic medical instruments, such as various types of ultrasonic scalpels. The ultrasonic medical instruments include vibration sources (such as ultrasound transducers, which can be used as handles for the ultrasonic medical instruments) and actuation components (configured to amplify vibrations generated by the vibration sources for surgical operations). By providing a gap between the portion of the medical instrument and the channel, it is possible to prevent the illumination apparatus from affecting the operation of the actuation component of the ultrasonic medical instrument, and it is also possible to prevent vibration of the actuation component from affecting an illumination effect of the illumination apparatus.

### BRIEF DESCRIPTION OF THE DRAWINGS

In the accompanying drawings, the same reference signs denote the same or similar components or elements throughout a plurality of accompanying drawings unless otherwise specified. These accompanying drawings are not necessarily drawn to scale. It should be understood that these accompanying drawings depict only some embodiments according to the present disclosure herein and are not to be construed as limiting the scope of the present disclosure.
Fig. 1 is a perspective view of an illumination apparatus according to a first embodiment of the present disclosure;
Fig. 2 is an exploded view of the illumination apparatus shown in Fig. 1;
Fig. 3 is a perspective view of an illumination apparatus according to a second embodiment of the present disclosure;
Fig. 4 is an exploded view of the illumination apparatus shown in Fig. 3;
Fig. 5 is a perspective view of an illumination apparatus according to a third embodiment of the present disclosure;
Fig. 6 is an exploded view of the illumination apparatus shown in Fig. 5;
Fig. 7 shows a perspective view of an illumination apparatus according to the present disclosure connected to a medical instrument; and
Fig. 8 shows a perspective view of an illumination apparatus according to the present disclosure connected to a further medical instrument.

### DETAILED DESCRIPTION OF EMBODIMENTS

Only some exemplary embodiments will be briefly described below. As can be appreciated by those skilled in the art, the described embodiments can be modified in various ways without departing from the spirit or scope of the present disclosure. Accordingly, the accompanying drawings and the description are considered illustrative in nature rather than limited.

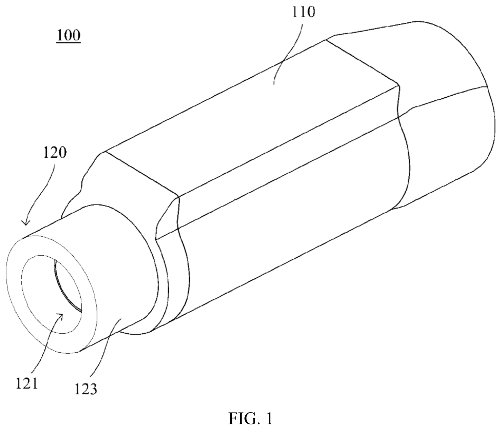

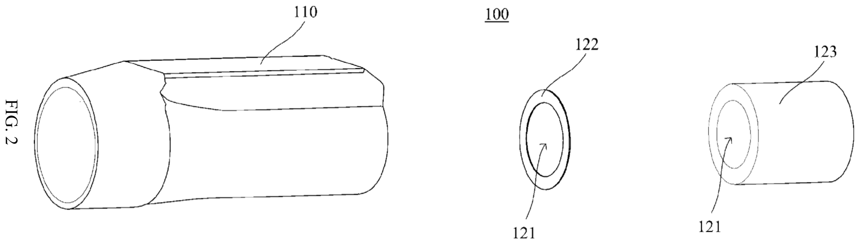

Fig. 1 is a perspective view of an illumination apparatus according to a first embodiment of the present disclosure; and Fig. 2 is an exploded view of the illumination apparatus shown in Fig. 1. As shown in Figs. 1 and 2, an illumination apparatus 100 for a medical instrument (not shown) according to some embodiments of the present disclosure includes: a housing component 110 provided with an accommodating space that runs along a longitudinal axis of the housing component 110; and an illumination assembly 120 disposed within the accommodating space and provided with a channel 121 that runs along the longitudinal axis.

By applying the illumination apparatus 100 according to some embodiments of the present disclosure to a medical instrument, such as by securing the illumination apparatus 100 to the medical instrument, direct illumination can be provided to an operating site during operation of the medical instrument, thereby facilitating a clear view of the operating site by an operator of the medical instrument.

It should be understood that "medical instrument" in the present disclosure may include any type of medical instruments, such as a scalpel, a surgical clamp, surgical forceps, an irrigation or suction tubing, etc. The scalpel may include an ultrasonic scalpel and an electrosurgical scalpel.

In some embodiments according to the present disclosure, the illumination assembly 120 may include: a light-emitting element 122; and a light-guiding element 123 disposed adjacent to the light-emitting element 122, the light-guiding element 123 being configured to guide light emitted from the light-emitting element 122 to the outside of the illumination apparatus 100; where the light-emitting element 122 and the light-guiding element 123 together define a channel 121.

In the above embodiments of the present disclosure, the light-emitting element 122 may be disposed abutting against the light-guiding element 123 or may be spaced apart from the light-guiding element 123 by a distance so long as the light-guiding element 123 is capable of guiding the light emitted from the light-emitting element 122 to the outside of the illumination apparatus 100.

In some embodiments according to the present disclosure, as shown in Fig. 2, the light-emitting element 122 may be annular, and the light-guiding element 123 may also be cylindrical, thus the light-emitting element 122 and the light-guiding element 123 can together define a channel 121 having a circular cross-section.

In some embodiments according to the present disclosure, the light-emitting element 122 includes any one or a combination of two or more of a halogen lamp, a light-emitting diode (LED) emitter, or an optical fiber.

In some embodiments according to the present disclosure, the light-guiding element 123 is transparent or translucent.

In some embodiments according to the present disclosure, the light-guiding element 123 is made of any one or more materials selected from polycarbonate, polyethylene terephthalate, polypropylene, and polymethyl methacrylate.

In some embodiments according to the present disclosure, the light-guiding element 123 further includes a light diffusion agent. By adding the light diffusion agent to the light-guiding element, an illumination range and/or uniformity of illumination of the illumination apparatus 100 can be expanded.

In some embodiments according to the present disclosure, the light-guiding element 123 extends by a predetermined distance from the accommodating space.

Since an outer dimension of the light-guiding element 123 is smaller than an outer dimension of the housing component 110, the light-guiding element 123 may be brought closer to the operating site of the medical instrument by arranging the light-guiding element 123 to extend by a predetermined distance from within the accommodating space, and it also avoids obstructing the view of the operator of the medical instrument as the housing component 110 having a larger outer dimension is close to the operating site of the medical instrument.

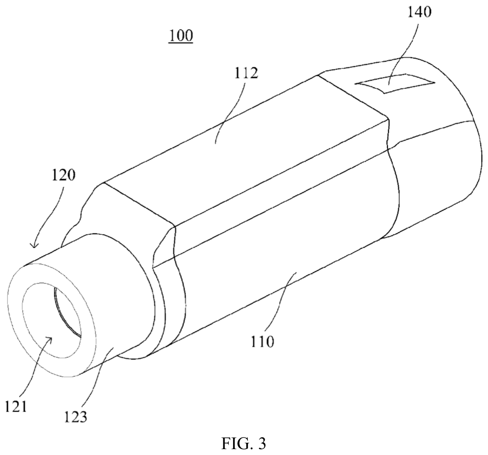

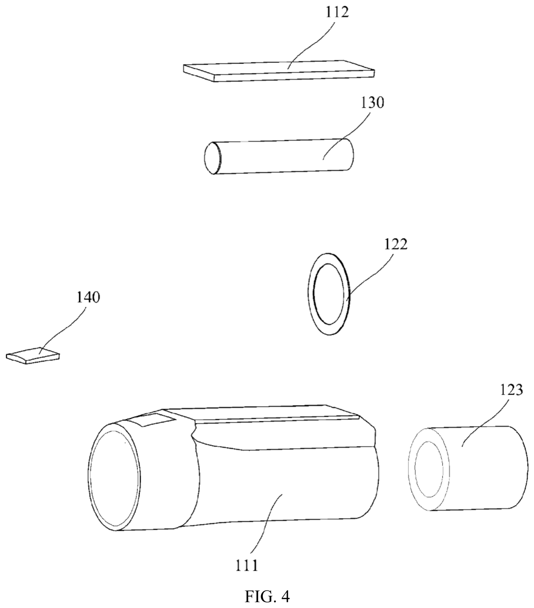

Fig. 3 is a perspective view of an illumination apparatus according to a second embodiment of the present disclosure; and Fig. 4 is an exploded view of the illumination apparatus shown in Fig. 3. The illumination apparatus 100 of the second embodiment shown in Figs. 3 and 4 differs from the illumination apparatus 100 of the first embodiment shown in Figs. 1 and 2 in that the illumination apparatus 100 of the second embodiment shown in Figs. 3 and 4 further includes other components.

Specifically, as shown in Fig. 4, the illumination apparatus 100 further includes a power supply 130 electrically connected to the illumination assembly 120.

It will be appreciated that the "power supply 130" herein may be a power supply fixedly connected to the illumination assembly 120, or may be a power supply detachably connected to the illumination assembly 120 (e.g., the power supply is an external power supply that is electrically connected to the illumination assembly by a wire). For example, the power supply may include a battery.

As shown in Fig. 4, the housing component 110 is provided with a power supply compartment 111 for accommodating the power supply 12 and a cover 112 for enclosing the power supply compartment 111.

In some embodiments according to the present disclosure, the cover 112 may hermetically enclose the power supply 130 in the power supply compartment.

Since the medical instrument may be in direct contact with a liquid such as a body fluid (such as blood) or an irrigation fluid during use, the illumination apparatus 100 may also be in direct contact with the body fluid (such as blood) or the irrigation fluid. By hermetically accommodating the power supply 130 in the power supply compartment 111, it is possible to avoid short-circuiting of the interior of the illumination apparatus 100 by these liquids.

In some embodiments according to the present disclosure, the illumination apparatus 100 further includes a switch 140 disposed in the housing component 110, the switch 140 being configured to selectively turn on and off an electrical connection between the power supply 130 and the illumination assembly 120.

By setting the above switch 140, it is possible to selectively turn on and off the electrical connection between the power supply 130 and the illumination assembly 120 during use of the medical instrument.

In some embodiments according to the present disclosure, the housing component 110 has a first end 112 and a second end 113 opposite each other, the light-guiding element 123 is disposed adjacent to the first end 112, and the switch 140 is disposed adjacent to the second end 113.

In order to better illuminate the operating site of the medical instrument, during use, the end of the light-guiding element 123 for illumination should be as close as possible to the operating site of the medical instrument, and for a procedure with a deeper wound, the end of the light-guiding element 123 for illumination may enter the wound. By placing the switch 140 as far away from the light-guiding element 123 as possible, the switch 140 can be easily operated.

In some embodiments according to the present disclosure, the switch 140 is a disposable switch.

In order to avoid possible infections caused by reuse, illustratively, the illumination apparatus 100 in the embodiments of the present disclosure may be a disposable illumination apparatus. In order to prevent the illumination apparatus 100 of the present disclosure from being undesirably reused, the switch 140 may be set as a disposable switch, i.e., the switch 140 cannot be turned off again once turned on, or the switch 140 cannot be turned on again once it is turned on and off once.

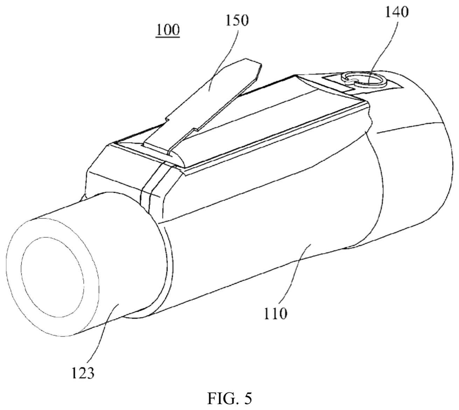

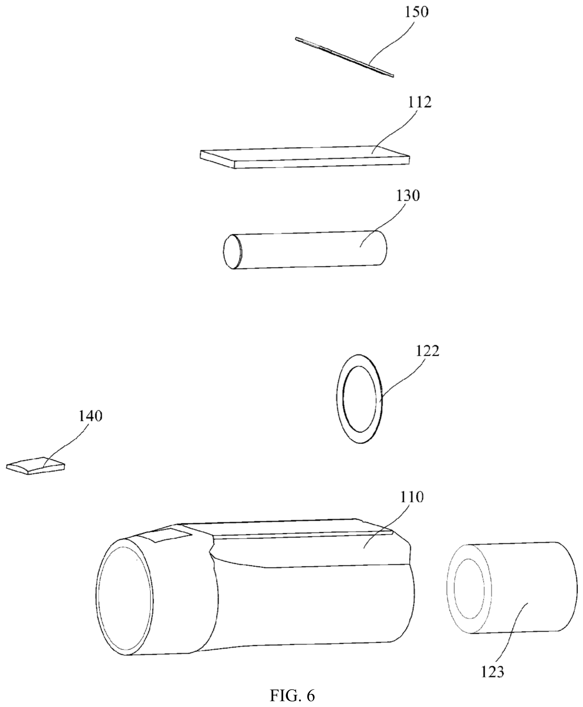

Fig. 5 is a perspective view of an illumination apparatus according to a third embodiment of the present disclosure; Fig. 6 is an exploded view of the illumination apparatus shown in Fig. 5; and the illumination apparatus 100 of the second embodiment shown in Figs. 5 and 6 differs from the illumination apparatus 100 of the first embodiment shown in Figs. 3 and 4 in that the illumination apparatus 100 of the second embodiment shown in Figs. 5 and 6 further includes other components.

Specifically, as shown in Figs. 5 and 6, the illumination apparatus 100 further includes a disposable insulation member 150 that is configured to disconnect the electrical connection between the power supply 130 and the illumination assembly 120.

In the above embodiments of the present disclosure, the disposable insulation member 150 described above can function as a switch, i.e., by removing the insulation member 150, the electrical connection between the power supply 130 and the illumination assembly 120 can be established, and the illumination apparatus 100 is thus used for illumination. Since the insulation member 150 is disposable, that is, once the insulation member 150 is removed from the illumination apparatus 100, the insulation member 150 cannot be used again to disconnect the electrical connection between the power supply 130 and the illumination assembly 120, the insulation member 150 also prevents the illumination apparatus 100 from being reused.

In the case where the illumination apparatus 100 of the present disclosure is itself provided with a switch 140 (such as the embodiment shown in Figs. 3 and 4) that selectively turns on and off the electrical connection between the power supply 130 and the illumination assembly 120, the insulation member 150 may be configured to prevent the illumination apparatus 100 from inadvertently touching the switch 140 during transport or use to establish an electrical connection between the power supply 130 and the illumination assembly 120. That is, before the disposable insulation member 150 is removed, even if the switch is on, the electrical connection between the power supply 130 and the illumination assembly 120 is also disconnected by the insulation member 150.

In some embodiments according to the present disclosure, the housing component 110 includes a connection (not shown) for detachably securing the illumination apparatus 100 to a medical instrument.

The above embodiments of the present disclosure are particularly applicable to reusable medical instruments. That is, after the medical instrument is used, the illumination apparatus 100 can be removed from the medical instrument to facilitate operations such as disinfection of the medical instrument.

According to another aspect of the present disclosure, some embodiments of the present disclosure provide a medical instrument kit, including: a medical instrument; and an illumination apparatus 100 according to any of the above embodiments.

It should be understood that according to the medical instrument kit of the above embodiments of the present disclosure, the illumination apparatus 100 may be fixedly disposed on the medical instrument, the illumination apparatus 100 and the medical instrument may be disposed separate from each other, and the illumination apparatus 100 is fixed to the medical instrument only when the medical instrument is in use.

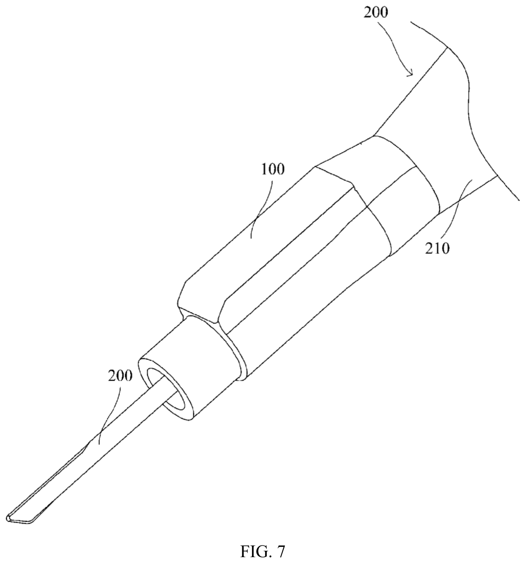

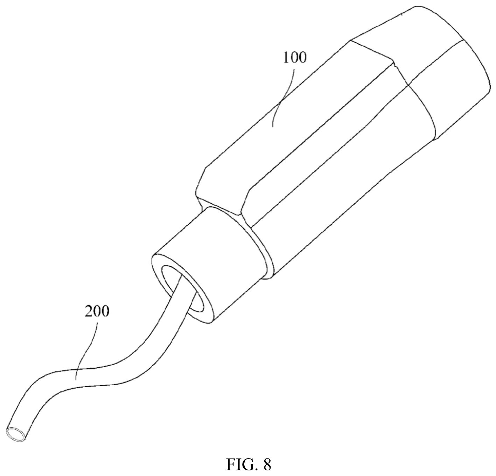

Fig. 7 shows a perspective view of an illumination apparatus 100 according to the present disclosure connected to a medical instrument 200. Fig. 8 shows a perspective view of an illumination apparatus 100 according to the present disclosure connected to a further medical instrument 200. In the embodiments shown in Figs. 7 and 8, a portion of the medical instrument 200 is accommodated within a channel of the illumination apparatus 100. In the embodiment shown in Fig. 7, the medical instrument 200 may be various types of scalpels. In the embodiment shown in Fig. 8, the medical instrument 200 may be various types of tubings, such as an irrigation tubing, a suction tubing, etc.

The illumination apparatus 100 is configured to illuminate an operating site of the medical instrument 200 so that an operator of the medical instrument 200 can clearly see the operating site. Thus, illustratively, the illumination apparatus 100 is disposed on the medical instrument 200 in proximity to the operating site of the medical instrument 200 to better illuminate the operating site of the medical instrument 200. As the portion of the medical instrument 200 is accommodated within the channel of the illumination apparatus 100, certain components of the medical instrument 200 can be prevented from blocking light emitted by the illumination apparatus 100, while 360-degree omnidirectional illumination around the medical instrument 200 is also provided.

In some embodiments according to the present disclosure, the illumination apparatus 100 is detachably fixed to the medical instrument 200.

As mentioned above, the above embodiments of the present disclosure are particularly applicable to reusable medical instruments 200. That is, after the medical instrument 200 is used, the illumination apparatus 100 can be removed from the medical instrument 200 to facilitate operations such as disinfection of the medical instrument 200.

In some embodiments according to the present disclosure, as shown in Fig. 7, the medical instrument 200 includes a handle 210, and the illumination apparatus 100 is detachably connected to the handle 210.

The above embodiments of the present disclosure are particularly applicable to ultrasonic medical instruments, such as various types of ultrasonic scalpels. The ultrasonic medical instruments include vibration sources (such as ultrasound transducers, which can be used as handles for the ultrasonic medical instruments) and actuation components (configured to amplify vibrations generated by the vibration sources for surgical operations). By securing the illumination apparatus 100 to the handle 210, it is possible to prevent the illumination apparatus 100 from affecting the operation of the actuation component of the ultrasonic medical instrument, and it is also possible to prevent vibration of the actuation component from affecting an illumination effect of the illumination apparatus 100.

In some embodiments according to the present disclosure, the illumination apparatus 100 is detachably connected to the handle 210 by a threaded connection or a snap connection or an interference fit.

In some embodiments according to the present disclosure, the illumination apparatus 100 is detachably fixed to the medical instrument 200 by an interference fit of the portion of the medical instrument 200 with the channel 121 of the illumination assembly 120.

In some embodiments according to the present disclosure, a gap is provided between the portion of the medical instrument 200 and the channel 121.

The above embodiments of the present disclosure are also particularly applicable to ultrasonic medical instruments, such as various types of ultrasonic scalpels. The ultrasonic medical instruments include vibration sources (such as ultrasound transducers, which can be used as handles for the ultrasonic medical instruments) and actuation components (configured to amplify vibrations generated by the vibration sources for surgical operations). By providing a gap between the portion of the medical instrument 200 and the channel 121, it is possible to prevent the illumination apparatus 100 from affecting the operation of the actuation component of the ultrasonic medical instrument, and it is also possible to prevent vibration of the actuation component from affecting an illumination effect of the illumination apparatus 100.

This description provides many different embodiments or examples that can be used to implement the present disclosure. It should be understood that these various embodiments or examples are purely illustrative and are not intended to limit the scope of protection of the present disclosure in any way. On the basis of the disclosure of the description of the present disclosure, those skilled in the art will be able to conceive of various changes or substitutions. Any changes or substitutions shall fall within the scope of protection of the present disclosure. Therefore, the scope of protection of the present disclosure shall be subject to the scope of protection of the claims.

## Claims

1. An illumination apparatus for a medical instrument, the illumination apparatus comprising:
a housing component provided with an accommodating space that runs along a longitudinal axis of the housing component; and
an illumination assembly disposed within the accommodating space and provided with a channel that runs along the longitudinal axis.

2. The illumination apparatus according to claim 1, wherein the illumination assembly comprises:
a light-emitting element; and
a light-guiding element disposed adjacent to the light-emitting element, the light-guiding element being configured to guide light emitted from the light-emitting element to the outside of the illumination apparatus;
wherein the light-emitting element and the light-guiding element together define the channel.

3. The illumination apparatus according to claim 2, wherein the light-emitting element is annular, the light-guiding element is cylindrical, and the light-emitting element and the light-guiding element together define the channel having a circular cross-section.

4. The illumination apparatus according to claim 2 or 3, wherein the light-emitting element comprises any one or a combination of two or more of a halogen lamp, an LED emitter, or an optical fiber.

5. The illumination apparatus according to any one of claims 2 to 4, wherein the light-guiding element is transparent or translucent.

6. The illumination apparatus according to any one of claims 2 to 5, wherein the light-guiding element is made of any one or more materials selected from polycarbonate, polyethylene terephthalate, polypropylene, and polymethyl methacrylate.

7. The illumination apparatus according to claim 6, wherein the light-guiding element further comprises a light diffusion agent.

8. The illumination apparatus according to any one of claims 2 to 7, wherein the light-guiding element extends by a predetermined distance from the accommodating space.

9. The illumination apparatus according to any one of claims 1 to 8, wherein the illumination apparatus further comprises a power supply electrically connected to the illumination assembly.

10. The illumination apparatus according to claim 9, wherein the housing component is provided with a power supply compartment for accommodating the power supply and a cover for enclosing the power supply compartment.

11. The illumination apparatus according to claim 10, wherein the cover hermetically encloses the power supply in the power supply compartment.

12. The illumination apparatus according to any one of claims 9 to 11, wherein the illumination apparatus further comprises a switch disposed in the housing component, the switch being configured to selectively turn on and off an electrical connection between the power supply and the illumination assembly.

13. The illumination apparatus according to claim 12, wherein the illumination apparatus is an illumination apparatus according to any one of claims 2 to 8, the housing component has a first end and a second end opposite each other, the light-guiding element is disposed adjacent to the first end, and the switch is disposed adjacent to the second end.

14. The illumination apparatus according to claim 12 or 13, wherein the switch is a disposable switch.

15. The illumination apparatus according to any one of claims 10 to 14, wherein the illumination apparatus further comprises a disposable insulation member that is configured to disconnect an electrical connection between the power supply and the illumination assembly.

16. The illumination apparatus according to any one of claims 1 to 14, wherein the housing component comprises a connection for detachably securing the illumination apparatus to the medical instrument.

17. A medical instrument kit, comprising:
a medical instrument; and
an illumination apparatus according to any one of claims 1 to 16.

18. The medical instrument kit according to claim 17, wherein a portion of the medical instrument is accommodated within a channel of an illumination assembly.

19. The medical instrument kit according to claim 18, wherein the illumination apparatus is detachably fixed to the medical instrument.

20. The medical instrument kit according to claim 18 or 19, wherein the medical instrument comprises a handle, and the illumination apparatus is detachably connected to the handle.

21. The medical instrument kit according to claim 20, wherein the illumination apparatus is detachably connected to the handle by a threaded connection or a snap connection or an interference fit.

22. The medical instrument kit according to any one of claims 18 to 21, wherein the illumination apparatus is detachably fixed to the medical instrument by an interference fit of the portion of the medical instrument with the channel of the illumination assembly.

23. The medical instrument kit according to any one of claims 20 to 22, wherein a gap is provided between the portion of the medical instrument and the channel.
